**Europäisches Patentamt**

**European Patent Office**

**Office européen des brevets**

(11) Veröffentlichungsnummer: **0 198 933**
**B1**

(12) **EUROPÄISCHE PATENTSCHRIFT**

(45) Veröffentlichungstag der Patentschrift:
23.08.89

(21) Anmeldenummer: **85104918.9**

(22) Anmeldetag: **23.04.85**

(51) Int. Cl.⁴: **F 16 C 11/08,** F 16 C 11/10,
F 16 M 11/14, A 61 B 19/00,
G 02 B 21/24

(54) Lasthemmendes Gelenk, insbesondere für Operations mikroskope, und für diese ausgebildete Aufhängung.

(30) Priorität: **18.04.85 DE 8511531 U**

(43) Veröffentlichungstag der Anmeldung:
**29.10.86 Patentblatt 86/44**

(45) Bekanntmachung des Hinweises auf die Patenterteilung:
**23.08.89 Patentblatt 89/34**

(84) Benannte Vertragsstaaten:
**AT BE CH DE FR GB IT LI LU NL SE**

(56) Entgegenhaltungen:
**DE-U-1 891 958**
**FR-A-1 064 065**
**FR-A-1 373 630**
**GB-A-2 074 337**
**US-A-2 516 428**

(73) Patentinhaber: **J.D. Möller Optische Werke GmbH,**
**Rosengarten 10, D-2000 Wedel (DE)**

(72) Erfinder: **Twisselmann, Lorenz, Dr.- Ing.,**
**Hudenkamp 4, D-2061 Prisdorf (DE)**

(74) Vertreter: **Patentanwälte Dipl.- Ing. J. Richter**
**Dipl.- Ing. F. Werdermann, Neuer Wall 10, D-2000**
**Hamburg 36 (DE)**

**Beschreibung**

Die Erfindung betrifft ein lasthemmendes Gelenk, insbesondere für Operationsmikroskope, das aus einer in einem Gehäuse gelagerten Gelenkkugel mit einem angeformten Gelenkzapfen oder -stange besteht, die in ihrer Lagerung durch mechanische Klemmelemente in ihrer freien Beweglichkeit hemmbar ist, die zur Aufhebung einer Selbsthemmung und zur Erzielung einer freien Beweglichkeit der Gelenkkugel mittels Einrichtungen von außen außer Eingriff mit der Gelenkkugel bringbar sind, wobei Federn vorgesehen sind, die die mechanischen Klemmelemente in Eingriff mit der Gelenkkugel zur Verhinderung der freien Beweglichkeit derselben bringen.

Ein derartiges lasthemmendes Gelenk ist aus der GB-A-2 074 337 bekannt. Bei diesem lasthemmenden Gelenk wirken die mechanischen Klemmelemente selbsthemmend, wobei sie mittels einer fluidbetätigten Einrichtung außer Eingriff mit der Gelenkkugel bringbar sind. Hinzu kommt, daß eine Anzahl von Scheibenfedern vorgesehen ist, die die mechanischen Klemmelemente in Eingriff mit der Gelenkkugel bringen, um deren freie Beweglichkeit zu verhindern. Der Einsatzbereich dieses lasthemmenden Gelenkes ist jedoch beschränkt, da eine fluidbetätigte Einrichtung vorgesehen ist, um die Klemmelemente mittels dieser fluidbetätigten Einrichtung außer Eingriff mit der Gelenkkugel zu bringen. Als Fludium wird Druckluft eingesetzt, um die Klemmelemente mit der Gelenkkugel außer Eingriff bringen zu können. Das lasthemmende Gelenk ist daher nur überall dort einsetzbar, wo auch Druckluft zur Verfügung steht, was nicht immer der Fall ist. Hinzu kommt, daß durch die Verwendung von Druckluft besondere Dichtungseinrichtungen vorgesehen sein müssen; werden diese im Laufe der Zeit undicht, dann besteht die Gefahr, daß Druckluft an den undichten Stellen entweicht und nicht mehr ausreichend zur Verfügung steht, um die Klemmelemente mit der Gelenkkugel außer Eingriff zu bringen, ein Umstand, der es nicht erlaubt, derartige lasthemmende Gelenke bei Operationsmikroskopen einzusetzen. Außerdem sind derartige lasthemmende Gelenke, bei denen u.a. Druckluft eingesetzt wird, nicht so kostengünstig herzustellen, als wenn ein lasthemmendes Gelenk ausschließlich aus mechanischen Teilen besteht.

Ferner ist aus der US-A-2 516 428 ein lasthemmendes Gelenk bekannt, das um eine Drehachse angeordnet und das mechanisch entriegelbar ist. Die Entriegelung erfolgt mittels in Führungen bewegbarer, federbeaufschlagter Kugeln, deren Bewegungsbereich vermittels Stellschrauben vorgebbar ist. Die Funktion dieses lasthemmenden Gelenkes ist nur um eine Achse wirksam.

Der Erfindung liegt die Aufgabe zugrunde, ein räumlich wirkendes, lasthemmendes Gelenk, insbesondere für Operationsmikroskope, zu schaffen, welches ohne großen technischen Aufwand wirtschaftlich und ausschließlich aus mechanischen Teilen herstellbar und darüber hinaus als Operationsmikroskopaufhängung einsetzbar ist.

Eine erste Lösung der Aufgabe sieht ein lasthemmendes Gelenk gemäß der eingangs genannten Art vor, bei dem die mechanischen Klemmelemente aus federbeaufschlagten und auf einem Ring aufsitzenden Kugeln bestehen, die in einem von der Gelenkkugel und einer in dem die Gelenkkugel aufnehmenden Gehäuse ausgebildeten zylindrischen Bohrung gebildeten, konischen Ringspalt angeordnet sind, bei dem der Tangens des Konuswinkels kleiner als die Reibzahl der beteiligten Werkstoffe aufeinander ist, so daß bei einer Drehung der Gelenkkugel die Kugeln auf der einen Seite in den konischen Ringspalt hineinrollend sind, wobei für die Aufhebung der Selbsthemmung ein Hebel vorgesehen ist, der über Stifte den Ring verschiebend ist, so daß die Kugeln von der die elastischen Kräfte bewirkenden Einrichtung, wie Federn od. dgl., unbeaufschlagt und nicht in den konischen Ringspalt eindrückbar sind.

Als zweite Lösung der Aufgabe sieht die Erfindung ein lasthemmendes Gelenk der eingangs genannten Art vor, bei dem die mechanischen Klemmelemente zur Erzielung einer geringeren Flächenpressung aus die Selbsthemmung bewirkenden Körpern, wie Stifte oder Scheibensegmente oder Segmente eines radial geschlitzten Federringes, bestehen, wobei zur Erzielung der Selbsthemmung die auf einem Ring im Innenraum des die Gelenkkugel aufnehmenden Gehäuses liegenden mechanischen Elemente an der Gelenkkugelfläche anliegend und bei einer Drehung der Gelenkkugel an dieser ihrer Seite mitnehmend und an der anderen Seite der Gelenkkugel sich an einer festen Anlage abstützend sind, wobei die Länge zwischen dem Punkt auf der Gelenkkugel und dem Abstützpunkt an dem Gehäuse mit einer Steigung zunehmend ist, deren Tangens kleiner als die Reibzahl ist, und wobei zur Aufhebung der Selbsthemmung die Klemmelemente von außen über Stifte und den Ring gegen die die Stifte oder Scheibensegmente beaufschlagenden Federn außer Eingriff bringbar sind.

Bei einem derart ausgebildeten lasthemmenden Kugelgelenk wird die Selbsthemmung dadurch bewirkt, daß die Klemmelemente an der Gelenkkugelfläche anliegen und, soweit es sich um Klemmelemente in Form von Stiften oder Scheibensegmenten handelt, bei der Drehung der Gelenkkugel an dieser Seite mitgenommen werden, sich jedoch an der anderen Seite an einer festen Anlage abstützen, wobei dabei die Länge zwischen dem Punkt auf der Gelenkkugel und dem Abstützpunkt mit einer Steigung zunimmt, deren Tangens kleiner als die Reibzahl ist.

Das lasthemmende Kugelgelenk kommt zur Anwendung, wenn eine einmal eingestellte Lage eines Teiles oder Gerätes beibehalten werden soll und wenn andererseits bei Aufhebung der Lasthemmung eine leichte räumliche Verstellbarkeit möglich sein soll.

Das lasthemmende Kugelgelenk weist eine einfache Bauweise auf und ist wirtschaftlich herstellbar. Besonders geeignet ist die Verwendung des lasthemmenden Kugelgelenkes als Aufhängung für Operationsmikroskope, wobei dann die Betätigungsorgane für die Verriegelung der Selbsthemmung in der Nähe des Operationsfeldes neben dem Mikroskopobjektiv angebracht sind und gleichzeitig dazu dienen, das Mikroskop in die richtige Arbeitsstellung zu schwenken.

Vorteilhafte Ausgestaltungen der Erfindung sind in den Unteransprüchen gekennzeichnet, wobei eine weitere Lösung der gestellten Aufgabe in der Verwendung eines lasthemmenden Kugelgelenkes mit den in den Ansprüchen angegebenen Merkmalen als Aufhängung für ein Operationsmikroskop zu dessen Verschwenkbarkeit und Verdrehung in einem Bereich um beliebige Winkel in allen Richtungen bei Aufhebung der Selbsthemmung der Gelenkkugel und zur Feststellung des Operationsmikroskopes bei in Eingriff gebrachter Selbsthemmung besteht.

Im folgenden wird der Gegenstand der Erfindung in den Zeichnungen erläutert. Es zeigt

Fig. 1 teils in Ansicht, teils in einem senkrechten Schnitt ein lasthemmendes Kugelgelenk mit federbeaufschlagten Kugeln als Klemmelement,

Fig. 2 teils in Ansicht, teils in einem senkrechten Schnitt ein lasthemmendes Kugelgelenk mit auf die Gelenkkugel einwirkenden Stiften als Klemmelemente,

Fig. 3 in einer Ansicht von oben das Kugelgelenk nach Fig. 2,

Fig. 4 teils in Ansicht, teils in einem senkrechten Schnitt ein lasthemmendes Kugelgelenk mit auf die Gelenkkugel einwirkenden Scheibensegmenten als Klemmelemente,

Fig. 5 in einer Ansicht von oben das Kugelgelenk nach Fig. 4

Fig. 6 teils in Ansicht, teils in einem senkrechten Schnitt ein lasthemmendes Kugelgelenk mit einem auf die Gelenkkugel einseitig einwirkenden Klemmelement,

Fig. 7 einen senkrechten Schnitt gemäß Linie VI-VI in Fig. 6 und

Fig. 8 in einer Vorderansicht ein in einem selbsthemmenden Kugelgelenk aufgehängtes Operationsmikroskop.

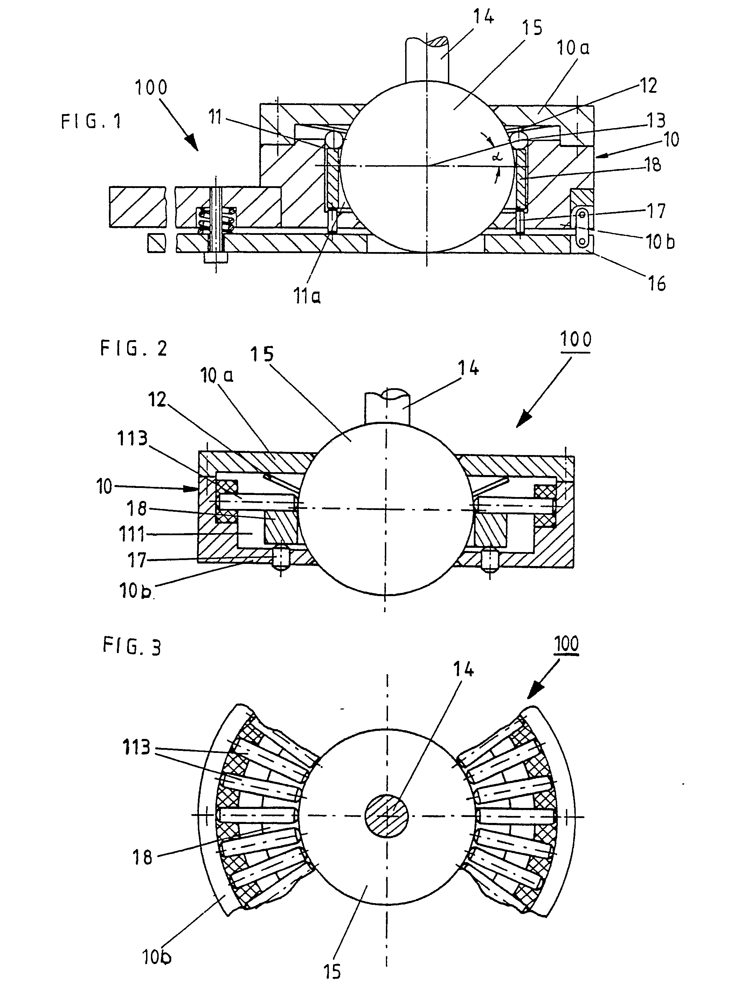

Bei dem in Fig. 1 dargestellten und mit 100 bezeichneten lasthemmenden Kugelgelenk ist in einem Gehäuse 10, welches aus den beiden Gehäuseteilen 10a, 10b besteht, eine Gelenkkugel 15 mit einem angeformten Gelenkzapfen bzw. -stange drehbar gelagert. Das Gehäuse 10 mit seinem Gehäuseteil 10b bildet mit der Gelenkkugel 15 einen konischen Ringspalt 11, in dem von mindestens einer Feder 12 Kugeln 13 soweit hineingedrückt werden, daß ihre Berührungspunkte mit der Gelenkkugel 15 unter einem Winkel $\alpha$ über dem Halbmesser liegen und tan

$\alpha \leqslant \mu$ ist. Das Gehäuseteil 10b des die Gelenkkugel 15 aufnehmenden Gehäuses 10 stellt das Bodenteil dar, das von dem Gehäuseteil 10a als obere Abdeckung abgedeckt ist. Die Kugeln 13, die einen kleinen Durchmesser gegenüber der Gelenkkugel 15 aufweisen und die Federn 12 stellen die mechanischen Klemmelemente für die Gelenkkugel 15 dar.

Bei einer Drehung der Gelenkkugel 15 werden die Kugeln 13 als Klemmelement auf einer Seite in den konischen Ringspalt 11 hineingerollt und führen zu einem Verklemmen der Gelenkkugel 15 in dem Gehäuse 10. Mittels dieser Kugeln 13 wird die Bewegung der Gelenkkugel 15 in ihrer Lagerung verhindert, was durch die Kugeln 13 als mechanisches Element aufgrund der erwirkten Selbsthemmung erreicht wird.

Die verwendeten Klemmelemente können von außen außer Eingriff gebracht werden, so daß dann keine Selbsthemmung mehr gegeben ist. Hierzu sind die Kugeln 13 auf einem im Innenraum des Gehäuses 10 angeordneten Ring 18 abgestützt, der über bei 17 angedeutete Stifte mit einem Betätigungshebel 16 in Verbindung steht, so daß mit diesem Betätigungshebel 16 über die Stifte 17 der Ring 18 verschiebbar ist, der die Kugeln 13 gegen die Kraft der Feder 12 aus dem Eingriff in dem konischen Ringspalt 11 drückt, so daß eine freie Beweglichkeit der Gelenkkugeln 15 erreicht wird.

Die Anordnung der Kugeln 13 erfolgt verteilt in dem konischen Ringspalt 11, der zwischen der Gelenkkugel 15 und der die Gelenkkugel 15 im Innenraum des Gehäuses 10 ausgebildeten zylindrischen Bohrung 11a gebildet wird. Die die Kugeln 13 beaufschlagenden Federn 12 stellen die elastische Kräfte bewirkenden Einrichtungen dar.

Da zwischen den Kugeln 13 in dem konischen Ringspalt 11 und der Gelenkkugel 15 hohe Flächenpressungen auftreten, ist die Verwendung andersartig ausgebildeter, die Flächenpressung verringernder Körper für die Erzielung der Selbsthemmung von Vorteil. Die in den Fig. 2, 3 und Fig. 4, 5 gezeigten Ausführungsbeispiele lasthemmender Kugelgelenke zeigen Möglichkeiten, bei denen rotationssymmetrisch zur Gelenkkugel 15 angeordnete Klemmkörper zur Selbsthemmung der Gelenkkugel 15 führen.

Das lasthemmende Kugelgelenk 100 gemäß Fig. 2 und 3 besteht ebenfalls aus einem Gehäuse 10 mit den beiden Gehäuseteilen 10a, 10b, von denen das Gehäuseteil 10b das Bodenteil und das Gehäuseteil 10a die obere Abdeckung bildet. Im Innenraum des Gehäuses 10 ist die Gelenkkugel 15 angeordnet. Außerdem ist im Innenraum des Gehäuses 10 ein Ringspalt 111 ausgebildet, der zur Aufnahme einer Anzahl von Stiften 113 dient, die sich auf ihrer der Gelenkkugel 15 abgewandten Seite an der Innenwandfläche des Gehäuses 10 abstützen und die mit ihren anderen freien Enden auf der Oberfläche der Gelenkkugel 15 anliegen und sich auf einem Ring 18 im Innenraum des Gehäuses abstützen, der mit durch die Gehäusewandung hindurchgeführten Stiften 17 in Wirkverbindung steht, die wiederum mit einem in Fig. 2 nicht dargestellten Betätigungshebel verbunden sind, der entsprechend dem Betätigungshebel 16 bei dem in Fig. 1 dargestellten lasthemmenden Kugelgelenk 100 ausgebildet ist.

Die Stifte 113 sind ebenfalls von Federn 12 beaufschlagt und bilden mit diesen zusammen die Klemmelemente. Außerdem sind die Stifte 113 radial, und zwar in einer Ebene liegend, d.h. auf dem Ring 18 liegend, um die Gelenkkugel 15 angeordnet.

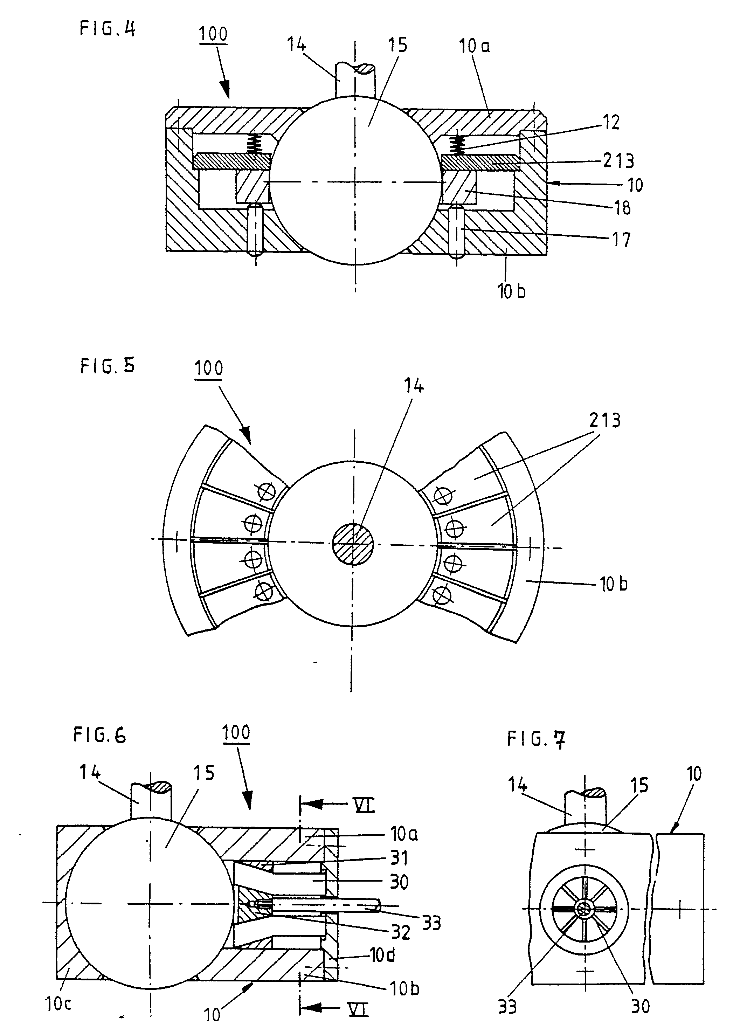

Bei der in Fig. 4 und 5 gezeigten Ausführungsform sind anstelle von Stiften 113 als Klemmelemente um die in dem Gehäuse 10 angeordnete Gelenkkugel 15 eine Anzahl von Scheibensegmenten 213 als Klemmkörper angeordnet, die entsprechend der Stifte 113 in gleicher Weise rotationssymmetrisch zur Gelenkkugel 15 angeordnet sind. Die Ausgestaltung des lasthemmenden Kugelgelenkes 100 gemäß Fig. 4 und 5 entspricht ansonsten der in Fig. 2 und 3 gezeigten Ausführungsform. Die Scheibensegmente 213 sind ebenfalls von Federn 12 beaufschlagt. Bei allen Ausführungsformen des lasthemmenden Kugelgelenkes entsprechend Fig. 1 und 2, 3 und 4, 5 ist es von Vorteil, wenn jeder Klemmkörper von einer Feder beaufschlagt ist.

Die Selbsthemmung unter Verwendung von Stiften 113 bzw. Scheibensegmenten 213 als Klemmelemente wird dadurch bewirkt, daß die Stifte bzw. Scheibensegmente an der Außenfläche der Gelenkkugel 15 anliegen und bei einer Drehung der Gelenkkugel an dieser Seite mitgenommen werden; sie stützen sich jedoch an der anderen Seite an einer festen Anlage, hier Gehäuseinnenwand, ab, wobei die Länge zwischen dem Punkt auf der Gelenkkugel 15 und dem Abstützpunkt mit einer Steigung zunimmt, deren Tangens kleiner als die Reibzahl ist.

Auch in den Fällen, in denen Stifte 113 oder Scheibensegmente 213 entsprechend Fig. 2, 3 und Fig. 4, 5 als Klemmelemente verwendet werden, wird die Selbsthemmung aufgehoben, wenn die Klemmelemente von außen über die Stifte 17 und den Ring 18 gegen die Federn 12 außer Eingriff gebracht werden.

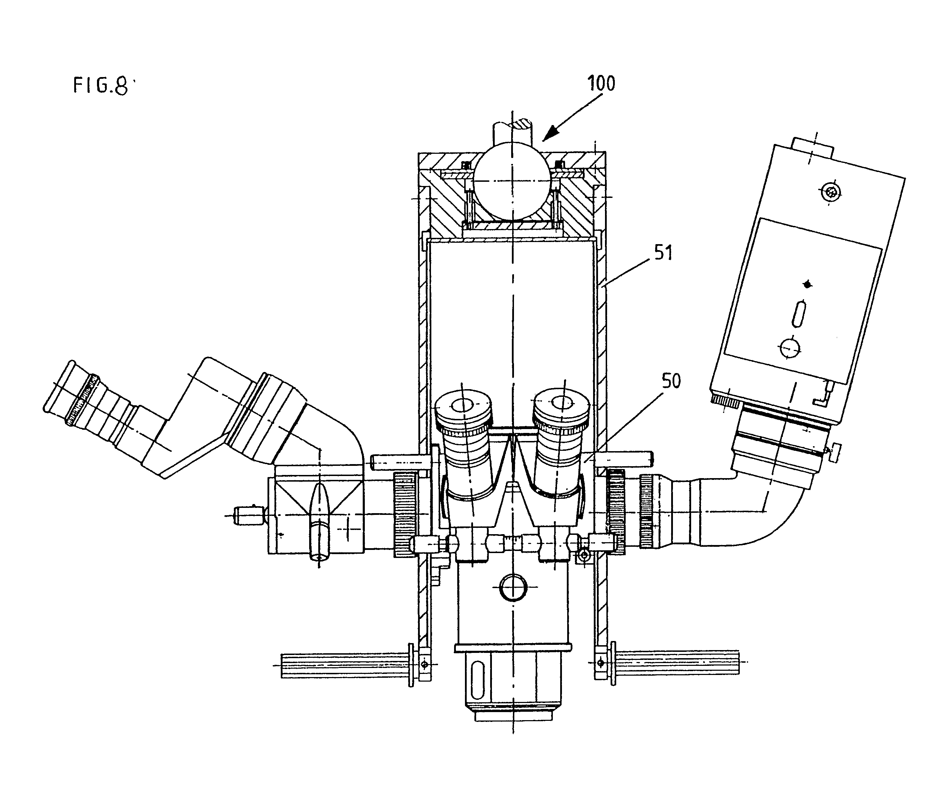

Bei der in Fig. 6 und 7 gezeigten Ausführungsform eines lasthemmenden Kugelgelenkes ist ebenfalls in einem Gehäuse 10 eine Gelenkkugel 15 angeordnet, wobei das Gehäuse 10 aus den beiden Teilen 10a und 10b besteht, die unter Ausbildung der Ausnehmung zur Aufnahme der Gelenkkugel 15 über das rückwärtige Teil 10c verbunden sind. In einer zylindrischen Ausnehmung in dem Gehäuse 10, die durch das Teil 10d abschließbar ist, ist mindestens ein rotationssymmetrischer Klemmkörper 30 angeordnet. Die rotationssymmetrischen Klemmkörper 30 wirken einseitig auf die in dem Gehäuse 10 drehbar gelagerte Gelenkkugel 15. Jeder Klemmkörper 30 kann mit einem Stellkörper mit einem Innenkonus 31 und einem Außenkonus 32 über eine bei 33 angedeutete Schubstange in und außer Eingriff gebracht werden, so daß die Selbsthemmung erzielt oder verhindert wird.

Ein Anwendungsbeispiel eines lasthemmenden Kugelgelenkes 100 ist in Fig. 8 dargestellt. Hier ist ein Operationsmikroskop 50 mit einer Mikroskopaufhängung 51 an einem derartigen lasthemmenden Kugelgelenk 100, wie voranstehend beschrieben und in Fig. 1 bis 7 dargestellt, aufgehängt. Die Betätigungsorgane, wie Betätigungshebel 16 und die Schubstange 33 für die Entriegelung der Selbsthemmung sind dann vorzugsweise in der Nähe des Operationsfeldes neben dem Mikroskopobjektiv angebracht und können gleichzeitig so ausgestaltet sein, daß sie dazu dienen, das Mikroskop in die richtige Arbeitsstellung zu schwenken.

**Patentansprüche**

1. Lasthemmendes Gelenk, insbesondere für Operationsmikroskope, das aus einer in einem Gehäuse (10) gelagerten Gelenkkugel (15) mit einem angeformten Gelenkzapfen oder -stange (14) besteht, die in ihrer Lagerung durch mechanische Klemmelemente in ihrer freien Beweglichkeit hemmbar ist, die zur Aufhebung einer Selbsthemmung und zur Erzielung einer freien Beweglichkeit der Gelenkkugel (15) mittels Einrichtungen von außen außer Eingriff mit der Gelenkkugel (15) bringbar sind, wobei Federn (12) vorgesehen sind, die die mechanischen Klemmelemente in Eingriff mit der Gelenkkugel (15) zur Verhinderung der freien Beweglichkeit derselben bringen, dadurch gekennzeichnet, daß die mechanischen Klemmelemente (12, 13) aus federbeaufschlagten (12) und auf einem Ring (18) aufsitzenden Kugeln (13) bestehen, die in einem von der Gelenkkugel (15) und einer in dem die Gelenkkugel (15) aufnehmenden Gehäuse (10) ausgebildeten zylindrischen Bohrung gebildeten konischen Ringspalt (11) angeordnet sind, bei dem der Tangens des Konuswinkels kleiner als die Reibzahl der beteiligten Werkstoffe aufeinander ist, so daß bei einer Drehung der Gelenkkugel (15) die Kugeln (13) auf der einen Seite in den konischen Ringspalt (11) hineinrollend sind, wobei für die Aufhebung der Selbsthemmung ein Hebel (16) vorgesehen ist, der über Stifte (17) den Ring (18) verschiebend ist, so daß die Kugeln (13) von der die elastischen Kräfte bewirkenden Einrichtung, wie Feder od. dgl. (12) unbeaufschlagt und nicht in den konischen Ringspalt (11) eindrückbar sind.

2. Lasthemmendes Gelenk, insbesondere für Operationsmikroskope, das aus einer in einem Gehäuse (10) gelagerten Gelenkkugel (15) mit einem angeformten Gelenkzapfen oder -stange (14) besteht, die in ihrer Lagerung durch mechanische Klemmelemente in ihrer freien Beweglichkeit hemmbar ist, die zur Aufhebung einer Selbsthemmung und zur Erzielung einer freien Beweglichkeit der Gelenkkugel (15) mittels Einrichtungen von außen außer Eingriff mit der Gelenkkugel (15) bringbar sind, wobei Federn (12) vorgesehen sind, die die mechanischen Klemmelemente in Eingriff mit der Gelenkkugel (15) zur Verhinderung er freien Beweglichkeit derselben bringen, dadurch gekennzeichnet, daß die mechanischen Klemmelemente (12, 113) zur Erzielung einer geringeren Flächenpressung aus die Selbsthemmung bewirkenden Körpern, wie Stifte (113) oder Scheibensegmente (213) oder Segmente eines radial geschlitzten Federringes bestehen, wobei zur Erzielung der Selbsthemmung die auf einem Ring (18) im Innenraum des die Gelenkkugel (15) aufnehmenden Gehäuses (10) liegenden mechanischen Elemente (113; 213) an der Gelenkkugelfläche anliegend und bei einer Drehung der Gelenkkugel (15) an dieser ihrer Seite mitnehmend und an der anderen Seite der Gelenkkugel (15) sich an einer festen Anlage abstützend sind, wobei die Länge zwischen dem Punkt auf der Gelenkkugel (15) und dem Abstützpunkt an dem Gehäuse mit einer Steigung zunehmend ist, deren Tangens kleiner als die Reibzahl ist, und daß zur Aufhebung der Selbsthemmung die Klemmelemente (113, 213) von außen über Stifte (17) und den Ring (18) gegen die die Stifte (113) oder Scheibensegmente (213) beaufschlagenden Federn (12) außer Eingriff bringbar sind.

3. Lasthemmendes Gelenk nach Anspruch 1 oder 2, dadurch gekennzeichnet, daß die die Selbsthemmung bewirkenden Klemmelemente (13; 113; 213) radial um die Gelenkkugel (15) angeordnet sind.

4. Lasthemmendes Gelenk nach einem der Ansprüche 1 bis 3, dadurch gekennzeichnet, daß die die Selbsthemmung bewirkenden Klemmelemente als rotationssymmetrische Klemmkörper (30) einseitig auf eine in einem Gehäuse (10) drehbar gelagerte Gelenkkugel (15) einwirkend und mit einem Stellkörper mit Innen- (30) und Außenkonus (32) über eine Schubstange (33) in und außer Eingriff bringbar sind.

5. Verwendung eines lasthemmenden Kugelgelenkes mit den Merkmalen der Ansprüche 1 bis 4 als Aufhängung für ein Operationsmikroskop zu dessen Verschwenkbarkeit und Verdrehung in einem Bereich um beliebige Winkel in allen Richtungen bei Aufhebung der Selbsthemmung der Gelenkkugel und zur Feststellung des Operationsmikroskopes bei in Eingriff gebrachter Selbsthemmung.

6. Verwendung nach Anspruch 5 einer in der Nähe des Operationsfeldes neben dem Mikroskopobjektiv angeordneten und als Betätigungsorgane zum Verschwenken des Operationsmikroskopes in die jeweilige Arbeitsstellung ausgebildeten Einrichtung zur Aufhebung der Selbsthemmung der Gelenkkugel.

## Claims

1. Load restraining pivot, particularly for operating microscopes, comprising a pivot ball (15) supported in a casing (10) with a pivot pin (14) constructed thereon which, in its support, can be restrained by mechanical clamping members in its free mobility which, for the cancellation of an automatic locking and for achieving a free mobility of the pivot ball (15), can, by means of devices from without, be brought out of engagement with the pivot ball (15), in which springs (12) are provided which bring the mechanical clamping members into engagement with the pivot ball (15) for preventing the free mobility of the same, characterized in that the mechanical clamping members (12, 13) consist of spheres (13) that are acted upon by springs (12) and seated upon a ring (18), which are disposed inside a conical annular gap (11) formed by the pivot ball (15) and inside a cylindrical bore constructed in the casing (10) accommodating the pivot ball (15), in which the tangent of the cone angle is smaller than the coefficient of friction of the materials involved on each other so that, when the pivot ball is rotated, the spheres (13) on the one side roll into the conical annular gap (11), in which, for the cancellation of the automatic locking, a lever (16) is provided which displaces the ring (18) across pins (17) so that the spheres (13) of the means giving rise to the elastic forces, such as a spring or the like (12) is not acted upon and cannot be pressed into the conical annular gap (11).

2. Load restraining pivot, particularly for operating microscopes, comprising a pivot ball (15) supported in a casing (10) with a pivot pin (14) constructed thereon which, in its support, can be restrained by mechanical clamping members in its free mobility which, for the cancellation of the automatic locking and for achieving a free mobility of the pivot ball (15) by means of devices from without, can be brought out of engagement with the pivot ball (15), in which springs (12) are provided which bring the mechanical clamping members into engagement with the pivot ball (15) so as to prevent the free mobility of the same, characterized in that the mechanical clamping members (12, 113), in order to achieve a lower surface pressure, consist of members bringing about the automatic locking, such as pins (113) or disk segments (213), or of segments of a radially slotted spring washer, in which, in order to achieve the automatic locking, the mechanical members (113, 213) reposing on a ring (18) within the interior of the casing (10) accommodating the pivot ball (15), bear upon the pivot ball surface and, when the pivot ball (15) is rotated, on this their side act in an entraining manner and, on the other side of the pivot ball (15), are supported on a firm contact surface, while the length between the point on the pivot ball (15) and the supporting point on the casing, increases with an inclination, the tangent of which is smaller than the coefficient of friction, and in that, in order to cancel the automatic locking, the clamping members (113; 213) can be made from without to disengage the springs (12) which act upon the pins (113) or disk segments (213).

3. Load restraining pivot according to claim 1, characterized in that the clamping members bringing about the automatic locking (13, 113, 213) are arranged in a radial manner around the pivot ball (15).

4. Load restraining pivot according to any of the claims 1 to 3, characterized in that the clamping members bring about the automatic locking are constructed so as to act unilaterally as axially symmetrical clamping members (30) upon a pivot ball (15) rotatably supported in a casing (10) and, by means of an adjusting member with an inner cone (30) and an outer cone (32), can be brought into and out of engagement with the aid of the connecting rod (33).

5. Utilization of a load-restraining pivot ball possessing the features of the claims 1 to 4 as suspension for an operating microscope for the pivotability and rotation of the latter within a range through any angle whatever and in all directions while cancelling the automatic locking of the pivot ball and for securing the operating microscope when the same is brought into automatic locking engagement.

6. Utilization according to the claim 5 of a device for cancelling the automatic locking of the pivot ball arranged in the proximity of the workfield next to the microscope objective and constructed as actuating members for pivoting the operating microscope into the respective working position.

**Revendications**

1. Pivot avec blocage de charge, en particulier pour microscopes d'opération, qui est composé par une bille articulée (15), positionnée dans un bâti (10) avec un tourillon articulé ou une tige articulée (14) faisant corps avec celle-ci, dont le positionnement peut être bloqué quant à sa mobilité libre par des éléments mécaniques de serrage, qui, pour supprimer un blocage automatique et pour obtenir la mobilité libre de la bille articulée (15) peuvent être désengrenés de la bille articulée (15) de l'extérieur par des dispositifs, des ressorts (12) étant prévus, qui mettent en prise les éléments mécaniques de serrage avec la bille articulée (15) pour empêcher la mobilité libre de celle-ci, caractérisé en ce que les éléments mécaniques de serrage (12, 13) consistent en des billes actionnées par ressort (12) et des billes (13) logées sur un anneau (18) qui sont placées dans une fente annulaire conique (11) formée par la bille articulée (15) et une forure cylindrique formée dans le bâti (10) qui loge la bille articulée (15), fente annulaire dont la tangente de l'angle conique est inférieure au coefficient de frottement des matières afférentes l'une sur l'autre, si bien que, lors d'une rotation de la bille articulée (15), les billes (13) entrent en roulant dans la fente annulaire conique (11) sur l'un des côtés, un levier (16), qui fait se déplacer l'anneau (18) par l'intermédiaire de chevilles (17), étant prévu pour supprimer le blocage automatique, si bien que les billes (13) ne sont pas poussées par le dispositif qui provoque les forces élastiques, tel que le ressort ou équivalent (12), et qu'elles ne peuvent pas être pressées dans la fente annulaire conique (11).

2. Pivot avec blocage de charge, en particulier pour microscopes d'opération, qui est composé par une bille articulée (15), positionnée dans un bâti (10) avec un tourillon articulé ou une tige articulée (14) faisant corps avec celle-ci, dont le positionnement peut être bloqué quant à sa mobilité libre par des éléments mécaniques de serrage, qui, pour supprimer un blocage automatique et pour obtenir la mobilité libre de la bille articulée (15) peuvent être désengrenés de la bille articulée (15) de l'extérieur par des dispositifs, des ressorts (12) étant prévus, qui mettent en prise les éléments mécaniques de serrage avec la bille articulée (15) pour empêcher la mobilité libre de celle-ci, caractérisé en ce que les éléments mécaniques de serrage (12, 113) pour la réalisation d'une pression superficielle plus faible sont constitués par des corps provoquant le blocage automatique, tels que des chevilles (113) ou des segments de disque (213) ou des segments d'une rondelle-resort fendue dans le sens radial, les éléments mécaniques (113, 213) qui, pour obtenir le blocage automatique, se trouvent placés sur un anneau (18) dans l'espace intérieur du bâti (10) qui loge la bille articulée (15) en étant adjacents à la surface de la bille articulée et entraînant, lors d'une rotation de la bille articulée (15) sur cette même face et s'appuyant sur un support fixe, sur l'autre face de la bille articulée (15), la longueur entre le point sur la bille articulée (15) et le point d'appui sur le bâti étant croissante avec un pas dont la tangente est inférieure au coefficient de frottement et que, pour supprimer le blocage automatique, les éléments de serrage (113, 213) peuvent être désengrenés de l'extérieur par l'intermédiaire de chevilles (17) et de l'anneau (18) à l'encontre des ressorts (12) qui poussent les chevilles (113) ou les segments de disque (213).

3. Pivot avec blocage de charge selon la revendication 1 ou 2, caractérisé en ce que les éléments de serrage (13; 113; 213) qui provoquent le blocage automatique sont placés radialement autour de la bille articulée (15).

4. Pivot avec blocage de charge selon l'une des revendications 1 à 3, caractérisé en ce que les éléments de serage provoquant le blocage automatique sont conçus comme des corps de serrage (30) à symétrie de révolution qui agissent d'un côté sur une bille articulée (15) positionnée de manière rotative dans un bâti (10), qu'ils peuvent s'engrener avec un organe de réglage avec cône intérieur (30) et cône extérieur (32) par l'intermédiaire d'une bielle (33) et qu'ils peuvent s'en désengrener.

5. Utilisation d'un pivot avec blocage de charge selon les caractéristiques de revendications 1 à 4 comme suspension pour un microscope d'opération pour le pivotement et la rotation de celui-ci dans une zone autour d'angles quelconques dans toutes les directions en supprimant le blocage automatique de la bille articulée et pour le blocage du microscope d'opération, le blocage automatique étant en prise.

6. Utilisation selon la revendication 5 d'un dispositif pour supprimer le blocage automatique de la bille articulée placé à proximité du champ opératoire près de l'objectif du microscope et formé comme un organe d'actionnement pour faire pivoter le microscope d'opération dans la position de travail respective.

FIG. 1

FIG. 2

FIG. 3

FIG. 4

FIG. 5

FIG. 6

FIG. 7

3

FIG.8

100

51

50

EP 0 198 933 B1